# EUROPÄISCHE PATENTSCHRIFT

(11) **EP 3 603 716 B1**
(45) Veröffentlichungstag und Bekanntmachung des Hinweises auf die Patenterteilung: **04.11.2020**
(21) Anmeldenummer: 19187512.9
(22) Anmeldetag: 22.07.2019
(51) Int. Cl.: A61M 16/04

(54) **BEATMUNGSVORRICHTUNG MIT ABSAUGLEITUNG**
VENTILATION DEVICE WITH A SUCTION PIPE
DISPOSITIF DE RESPIRATION POURVU DE CONDUITE D'EXTRACTION

(30) Priorität: 31.07.2018 DE 102018118582
(43) Veröffentlichungstag der Anmeldung: 05.02.2020
(73) Patentinhaber: Tracoe Medical GmbH, 55268 Nieder-Olm (DE)
(72) Erfinder: SCHNELL, Ralf, 63500 Seligenstadt (DE); DOFFING, Frank, 56288 Kastellaun (DE)
(74) Vertreter: WSL Patentanwälte Partnerschaft mbB

(56) Entgegenhaltungen:
- DE-A1- 19 734 821
- DE-A1-102014 109 001
- DE-C1- 4 208 912
- US-A- 4 840 173
- US-A1- 2012 000 471

## Beschreibung

Die vorliegende Erfindung betrifft eine Beatmungsvorrichtung, insbesondere eine Tracheostomiekanüle oder einen Endotrachealtubus, mit einem Kanülenrohr, welches eine Absaugleitung zum Absaugen von subglottischem Sekret aufweist, wobei die Absaugleitung einen distalen Einlass und einen proximalen Auslass hat.

Die Begriffe "distal" und "proximal" werden im Sinne der vorliegenden Erfindung aus der Sicht eines behandelnden Arztes verwendet, d.h. das proximale Ende des Kanülenrohrs ist außerhalb des Patientenkörpers angeordnet, während das distale Ende im eingebrachten Zustand der Beatmungsvorrichtung im Inneren der Trachea des Patienten liegt. "Subglottisches" Sekret bezeichnet das Sekret, das sich unterhalb der "Glottis", d. h. unterhalb des Kehlkopfes befindet. Häufig handelt es sich dabei um Speichel, der die Glottis passiert hat und sich oberhalb eines geblockten Cuffs ansammelt.

Beatmungsvorrichtungen werden in die Trachea eines Patienten eingeführt, um den Patienten mit Luft bzw. Atemgas zu versorgen. Das Kanülenrohr kann durch eine im Hals eines Patienten unterhalb des Kehlkopfbereichs künstlich erzeugte Öffnung (Tracheostoma) oder über den Mund und Rachenraum in die Trachea eines Patienten eingebracht werden. Das proximale Ende des Kanülenrohrs ist jeweils außerhalb des Patienten mit einem Beatmungsgerät verbindbar, während das distale Ende des Kanülenrohrs in der Trachea angeordnet ist. Das Kanülenrohr dient mit seinem zentralen Lumen der Durchleitung von Atemgas.

Zur Abdichtung des Kanülenrohrs in der Trachea weist das Kanülenrohr vieler herkömmlicher Beatmungsvorrichtungen auf seinem äußeren Umfang einen befüllbaren Cuff auf. Ein Cuff besteht üblicherweise aus einem aufblasbaren Schlauchabschnitt mit einer sehr geringen Wandstärke, dessen Endabschnitte außen auf dem Kanülenrohr anliegen und dort abgedichtet fixiert sind. Der Cuff hat mindestens in einem zentralen Abschnitt gegenüber dem Kanülenrohr ein deutliches Übermaß oder ist auf ein solches Übermaß aufblasbar. Nach dem Einführen des Kanülenrohrs in die Trachea wird der Cuff mit geringem Druck aufgeblasen, bis er mit der Wand der Trachea entlang seines Umfangs abdichtend, jedoch mit nur geringem Druck in Kontakt kommt.

Zusätzlich soll ein solcher an der Wand der Trachea anliegender Cuff verhindern, dass Sekret aus dem Mundraum an dem Kanülenrohr vorbei und in die Bronchien eines mit der Beatmungsvorrichtung versorgten Patienten gelangt. Da Sekret häufig Bakterien, Pilze oder Viren enthält, könnte dies ansonsten zu Infektionen in den Bronchien, wie beispielsweise "Ventilation assoziierten Pneumonien (VAP)" (beatmungsbedingten Lungenentzündungen) führen. Aufgrund des den Querschnitt der Trachea außerhalb des Kanülenrohres blockierenden Cuffs staut sich Sekret oberhalb des Cuffs im subglottischen Bereich, das heißt in dem Bereich zwischen Glottis (Kehlkopf) und Cuff.

Dies verursacht Probleme. Einerseits besteht das Risiko, dass das aufgestaute Sekret am Cuff vorbei in die Bronchien gelangt, wenn beispielsweise aufgrund von Faltenbildung, eines zu geringen Cuffdrucks oder durch aktive oder passive Bewegungen des Patienten eine ausreichende Abdichtung nicht aufrechterhalten wird. Andererseits kann sich das Sekret soweit aufstauen, dass es aus dem Tracheostoma am Hals herausläuft. Das feuchte Milieu führt in Kombination mit Bakterien und den im Speichel befindlichen Fermenten häufig zu schwerwiegenden Entzündungen im Bereich des Stomas.

Um Sekret aus dem erwähnten subglottischen Bereich zu entfernen, weist das Kanülenrohr eine in den subglottischen Bereich hineinreichende Absaugleitung auf. Der distale Einlass der Absaugleitung befindet sich im Bereich des proximalen Endes des Cuffs und der proximale Auslass der Absaugleitung befindet sich außerhalb des Patienten. Mithilfe einer Spritze oder eines speziell dafür vorgesehenen elektronischen Absauggerätes lässt sich das Sekret mithilfe der Absaugleitung aus dem subglottischen Bereich absaugen.

Die Absaugleitung kann auch unabhängig davon, ob ein Cuff vorhanden ist, für die Absaugung von Sekret aus der Umgebung des Kanülenrohrs verwendet werden.

Das Lumen der Beatmungsvorrichtung sollte möglichst groß sein, um den Atemwiderstand zu reduzieren. Damit das Kanülenrohr innerhalb der Trachea ausreichend Platz hat, sollte andererseits der Außendurchmesser des Kanülenrohrs idealerweise weniger als 70 % des Innendurchmessers der Trachea betragen. Somit kann der Innendurchmesser der Absaugleitung nicht beliebig groß gewählt werden. Bei herkömmlichen Beatmungsvorrichtungen lassen sich daher vermehrt Verstopfungen der Absaugleitung beobachten. Dies kann insbesondere bei sehr zähflüssigem Sekret passieren. Auch können feste Bestandteile im Sekret, wie z.B. Nahrungsreste oder verkrustetes Sekret, die Absaugleitung verstopfen. Insbesondere die Kombination aus zähflüssigem Sekret und festen Bestandteilen im Sekret ist sehr kritisch.

Des Weiteren kann es vorkommen, dass der distale Einlass der Absaugleitung während der Absaugung mit der Schleimhaut der Trachea in Kontakt kommt und sich an der Trachealwand festsaugt. In diesem Fall kann ebenfalls kein Sekret abgesaugt werden. Der Unterdruck am distalen Ende der Absaugleitung kann außerdem zu Schädigungen der Schleimhaut führen.

Vor diesem Hintergrund ist es eine Aufgabe der vorliegenden Erfindung eine Beatmungsvorrichtung mit einer Absaugleitung bereitzustellen, die eine verbesserte Absaugung von Sekret ermöglicht.

Erfindungsgemäß wird die Aufgabe durch eine Beatmungsvorrichtung der eingangs genannten Art gelöst, bei welcher die Absaugleitung im Abstand von dem Einlass und dem Auslass mindestens eine Engstelle mit lokal minimalem Querschnitt aufweist, von welcher aus sich der Querschnitt sowohl in proximaler als auch distaler Richtung trichterförmig oder stufenförmig erweitert.

Als Engstelle wird hier die axiale Position oder der axiale Abschnitt der Absaugleitung mit lokal minimalem Querschnitt (jenseits von Fertigungstoleranzen) bezeichnet, wobei der minimale Querschnitt, ausgehend von der Engstelle zu den proximalen und distalen Bezugsbereichen hin allmählich und zweckmäßigerweise im Wesentlichen kontinuierlich zunimmt.

Der auf die Absaugleitung bezogene Begriff "Querschnitt" bezeichnet in der vorliegenden Erfindungsbeschreibung und soweit nicht ausdrücklich anderes bestimmt ist, immer die freie innere Querschnittsfläche der Absaugleitung.

In einer Ausführungsform ist nur eine einzige solche Engstelle entlang der Absaugleitung vorgesehen, so dass der freie Gesamtquerschnitt der Absaugleitung an allen axialen Positionen außerhalb der Engstelle größer ist als an der Engstelle. In anderen Ausführungsformen können auch mehrere Engstellen vorgesehen sein, insbesondere auch solche zusätzlichen Engstellen die durch eine Aufteilung des Querschnitts in mehrere Teilquerschnitte hervorgerufen werden, wobei in solchen Fällen eine Erweiterung des Querschnitts, ausgehend von einer derartigen Engstelle, nicht mehr unbedingt trichterförmig sondern eher stufenförmig ist

An die Engstelle schließt sich in proximaler und distaler Richtung jeweils ein Leitungsabschnitt mit größer werdendem Querschnitt an. Der verringerte Querschnitt der Engstelle bewirkt, dass die Geschwindigkeit des durch die Absaugleitung fließenden Sekrets in diesem Bereich erhöht ist. Einzelne Tropfen oder Zusammenhaftungen von Sekret werden durch die erhöhte Geschwindigkeit in der Engstelle getrennt und zähflüssiges Sekret wird besser auseinandergerissen als im Falle eines gleichbleibenden Querschnitts. Das Sekret zeigt nach der Erkenntnis der Erfinder thixotrope Eigenschaften und kann daher durch die Erhöhung der Fließgeschwindigkeit insgesamt fließfähiger werden. Dadurch kann das Sekret auch nach dem Passieren der Engstelle leichter durch die Absaugleitung abgeführt werden.

In einer Ausführungsform beträgt der minimale Querschnitt an der Engstelle zwischen einschließlich 40 % und einschließlich 85 %, vorzugsweise zwischen einschließlich 50 % und einschließlich 75 %, des maximalen Querschnitts in den Bereichen proximal und distal von der Engstelle. Die Engstelle definiert genau die axiale Position des minimalen Querschnitts, kann sich aber mit einem konstanten minimalen Querschnitt auch über einen mehr oder weniger kurzen (zum Beispiel maximal 5 mm langen) axialen Leitungsabschnitt hinweg erstrecken.

Die maximalen Querschnitte in den Bereichen distal und proximal von der Engstelle können verschieden oder auch gleich groß sein. Im Zweifelsfall kann als Vergleichsmaßstab für den Querschnitt der Engstelle auch der maximale Querschnitt in einem Teil der proximalen und distalen Bereiche herangezogen werden, der durch einen Mindestabstand von jeweils zum Beispiel 5 mm von der Engstelle und von den Ein- und Auslassöffnungen definiert ist.

Der Querschnitt der Engstelle kann sich zu den distalen und proximalen Bezugsbereichen hin kontinuierlich oder in jeweils mehreren Stufen erweitern. Der maximale Querschnitt in den distalen und proximalen Bezugsbereichen kann beispielsweise das 2,5-fache bis 1,18-fache, insbesondere vorzugsweise auf das 2-fache bis 1,33-fache des minimalen Querschnittes der Engstelle betragen.

Ist der Querschnitt der Engstelle allerdings zu klein gewählt, kann die Absaugleitung verstopfen. Andererseits würde ein zu groß gewählter Querschnitt der Engstelle die Fließgeschwindigkeit in der Engstelle nur marginal erhöhen und zu keiner signifikant verbesserten Sekretabsaugung führen. Die vorstehend genannten Größenverhältnisse tragen diesen Umständen Rechnung.

Der minimale Querschnitt ist vorzugsweise auf eine axiale Position oder einen kurzen axialen Abschnitt von maximal 5 mm axiale Länge beschränkt, wobei diese Position oder dieser kurze axiale Abschnitt als Engstelle bezeichnet wird. Vorzugsweise erstreckt sich die Engstelle über eine axiale Länge von mindestens 0,01 mm. Die Engstelle kann beispielsweise eine axiale Länge zwischen 1 mm und 5 mm aufweisen. An die Engstelle schließen sich zum Beispiel erste und zweite Übergangsbereiche an, die ausgehend von der Engstelle trichterförmige oder stufenförmige Erweiterungen bilden, welche Teil der proximalen und distalen Bereiche sind. Diese Bereiche können im Abstand von der Engstelle einen konstanten oder auch variierenden Querschnitt haben, wobei der maximale Querschnitt im Allgemeinen weiter von der Engstelle entfernt liegt als von dem Einlass bzw. dem Auslass. Die proximalen und distalen Bereiche außerhalb der Engstelle einschließlich der konischen Übergangsbereiche haben zweckmäßigerweise jeweils eine axiale Länge von mindestens 3 % bis einschließlich 90 % der Länge der Absaugleitung die von dem Kanülenrohr unterlegt ist. Die vorzugsweise konischen ersten und zweiten Übergangsbereiche erstrecken sich zweckmäßigerweise über eine axiale Länge von mindestens 3 % bis einschließlich 60 % der Länge der Absaugleitung die von dem Kanülenrohr unterlegt ist. "Konisch" steht hier generell für eine allmähliche Verjüngung oder Erweiterung. Die von dem Kanülenrohr unterlegte Länge der Absaugleitung entspricht der axialen Länge der Absaugleitung, über die die Absaugleitung mit dem Kanülenrohr verbunden ist. Die axiale Länge der von dem Kanülenrohr unterlegten Absaugleitung ist demnach nie größer als die axiale Länge des Kanülenrohrs, selbst wenn die Absaugleitung als Schlauchabschnitt über das proximale Ende des Kanülenrohrs übersteht.

Zweckmäßigerweise kann die axiale Länge des distalen Bereichs vor der Engstelle einschließlich 3% bis einschließlich 30 % der Gesamtlänge der Kanüle betragen, während der proximale Bereich eine axiale Länge von einschließlich 60 bis einschließlich 90 % der Kanülenlänge haben kann. Die distalen und proximalen Bereiche können insbesondere mit den distalen und proximalen Übergangsbereichen identisch sein.

In einer Ausführungsform erweitert sich der Querschnitt der Absaugleitung, ausgehend von dem minimalen Querschnitt der Engstelle, in Richtung des distalen Einlasses in axialer Richtung des Kanülenrohrs über eine Länge von 0,5 cm bis 2,5 cm, vorzugsweise von 0,5 cm bis 1,5 cm, und/oder in Richtung des proximalen Auslasses in axialer Richtung des Kanülenrohrs über eine Länge von 2 cm bis 8 cm, vorzugsweise von 3 cm bis 6 cm, kontinuierlich oder stufenförmig .

In einer Ausführungsform ist der Abstand der Engstelle zum proximalen Auslass der Absaugleitung größer als der Abstand der Engstelle zum distalen Einlass der Absaugleitung. Vorzugsweise ist der Abstand von der Engstelle zu dem proximalen Auslass um das 2- bis 20-fache größer als der Abstand von der Engstelle zu dem distalen Einlass. Der Abstand von der Engstelle zu dem distalen Einlass kann bis einschließlich 50 mm, vorzugsweise bis einschließlich 20 mm betragen.

In einer Ausführungsform ist die Engstelle von dem distalen Einlass um mindestens 5 mm und von dem proximalen Auslass um mindestens 40 mm beanstandet.

Es hat sich als positiv erwiesen, den Querschnitt relativ bald hinter dem distalen Einlass zunächst zu verkleinern, um den Sekretfluss in der Absaugleitung zu Beginn zu beschleunigen. Die Engstelle kann mit seinem distalen Übergangsabschnitt praktisch unmittelbar an den distalen Einlass angrenzen. Der maximale Querschnitt des distalen Bereiches wird bei einer solchen Ausführungsform entweder durch den Öffnungsquerschnitt des distalen Einlasses gebildet oder aber durch den maximalen Querschnitt des distalen Bereiches, der vorzugsweise um mindestens 50% größer als der Querschnitt der Engstelle ist.

Die Abnahme des Querschnitts im distalen Bereich verläuft damit steiler als die Zunahme des Querschnitts im proximalen Bereich. Durch eine kontinuierliche Änderung der Querschnittsflächen wird der Sekretfluss sukzessive beschleunigt, sodass lose zusammenhängendes Agglomerat durch die erzeugten Scherkräfte zerteilt und die Viskosität des nichtnewtonschen Sekrets reduziert wird. Dies ermöglicht einen verbesserten Sekretfluss bei einem möglichst geringen Absaugvakuum.

Weiterhin hat sich gezeigt, dass die Form der distalen Einlassöffnung ebenfalls einen gewissen Einfluss auf die Handhabbarkeit der Absaugleitung hat. Damit größere Partikel nicht versehentlich durch den distalen Einlass in die Absaugleitung gelangen und möglicherweise die Engstelle blockieren, weist der distale Einlass in einer Ausführungsform mindestens zwei Öffnungen auf, die jeweils einen kleineren Öffnungsquerschnitt aufweisen als der maximale Querschnitt des distalen Bereiches, während die Summe der Öffnungsquerschnitte gleich groß wie oder auch größer als der maximale Querschnitt des distalen Bezugsbereiches sein kann. Vorzugsweise sind die zwei Öffnungen schlitzförmig.

Die mindestens zwei Öffnungen wirken ähnlich einem Sieb und verhindern, dass größere Partikel in die Absaugleitung gelangen. Zusätzlich reduziert die Aufteilung des distalen Einlasses auf mindestens zwei Öffnungen das Risiko von Schleimhautverletzungen. Im Gegensatz zu einer großen Einlassöffnung ist bei mindestens zwei vergleichsweise kleinen Einlassöffnungen die Gefahr verringert, dass die Schleimhaut der Trachea in die Absaugleitung hineingesaugt wird und somit weniger wahrscheinlich verletzt wird. Die zwei Einlassöffnungen können in einer Ausführungsform durch einen Steg voneinander getrennt sein, wobei die Öffnungen bevorzugt jeweils auf einer Ebene angeordnet sind, die einen Winkel von einschließlich 10° bis einschließlich 120° einschließen oder parallel zu der Längsachse der Absaugleitung angeordnet sind. Auf diese Weise kann verhindert werden, dass sich die mindestens zwei Öffnungen der Absaugleitung gleichzeitig an die Trachealwand bei der Absaugung ansaugen.

Die Innenfläche der Absaugleitung außerhalb der Engstelle kann mindestens einen Vorsprung aufweisen, der in das Lumen der Absaugleitung hineinragt, wobei der Vorsprung vorzugsweise ein sich in axialer Richtung der Absaugleitung erstreckender Steg oder Wandabschnitt ist, der bevorzugt im proximalen Bezugsbereich angeordnet ist. Alternativ oder zusätzlich zu einem sich in axialer Richtung erstreckendem Steg oder Wandabschnitt, kann der in das Lumen der Absaugleitung hineinragende Vorsprung im Querschnitt dreieckig, trapezförmig oder rechtwinklig ausgestaltet sein wobei Kanten vorzugsweise abgerundet sind. Ein solcher in das Lumen der Absaugleitung hineinragender Vorsprung ist ein Strömungshindernis, das den Sekretfluss in der Absaugleitung in zwei Ströme aufteilt und so hilft, das in der Absaugleitung befindliche Sekret auseinanderzureißen. Das auseinandergerissene Sekret kann einfacher transportiert werden, sodass der Vorsprung den Transport von Sekret insgesamt vereinfacht. Auch kann eine Mehrzahl von Vorsprüngen in einem sich wiederholenden Muster angeordnet sein.

Der in das Lumen der Absaugleitung hineinragende Vorsprung hat in einer Ausführungsform in axialer Richtung eine Länge, die 10% bis 30 % der Länge des Kanülenrohrs bzw. des auf dem Kanülenrohr befestigten Abschnittes der Absaugleitung entspricht.

Es hat sich als vorteilhaft erwiesen, wenn der mindestens eine Vorsprung proximal von der Engstelle angeordnet ist. Das zuvor in Längsrichtung gestreckte und auseinandergerissene Sekret wir dann nochmals in Querrichtung aufgeteilt. Das durch die Engstelle fließfähiger gemachte Sekret, bleibt so über eine längere Strecke zerkleinert bzw. fließfähig.

In einer Ausführungsform hat das Lumen der Absaugleitung eine in etwa sichelförmige Querschnittsform.

Um ein Anhaften von Sekret in der Absaugleitung zu vermeiden sowie den Transport durch die Absaugleitung zu vereinfachen, ist es in einer Ausführungsform vorgesehen, dass die der Querschnitt begrenzenden Wandflächen der Absaugleitung und/oder der mindestens eine in das Lumen der Absaugleitung hineinragende Vorsprung eine mittlere Oberflächenrauheit Ra von kleiner gleich 1,6 µm aufweisen.

In einer Ausführungsform wird das Lumen der Absaugleitung durch eine auf die Außenseite des Kanülenrohrs aufgesetzte Halbschale und die Außenseite des Kanülenrohrs begrenzt. Weil die Absaugleitung durch die Halbschale und die Außenseite des Kanülenrohrs gebildet wird, ist die Absaugleitung insgesamt platzsparender als Absaugrohre, die auf herkömmliche Weise mit dem Kanülenrohr verklebt sind. Der Innenquerschnitt der Absaugleitung kann dadurch insgesamt größer ausgestaltet sein, ohne dass der Außenquerschnitt des Kanülenrohrs, einschließlich der Absaugleitung, in gleichem Maße vergrößert ist. Kanülenrohr und Halbschale können aus dem gleichen Material oder unterschiedlichen Materialien hergestellt sein.

Prinzipiell könnte die Absaugleitung auch für die Zuleitung von Luft in die Trachea genutzt werden, um einem Patienten trotz eingeführter Beatmungsvorrichtung das Sprechen zu ermöglichen.

In einer Ausführungsform weist die Beatmungsvorrichtung zusätzlich zur Absaugleitung eine Luftleitung mit einer distalen Öffnung und einer proximalen Öffnung auf, wobei die distale Öffnung der Luftleitung proximal im Abstand zu dem distalen Einlass der Absaugleitung angeordnet ist. Beispielsweise beträgt der minimale lichte Abstand zwischen der distalen Öffnung der Luftleitung und dem distalen Einlass 5 mm bis 30 mm. Durch die proximal vom Einlass der Absaugleitung angeordneten Öffnung der Luftleitung wird die durch die Luftleitung zugeführte Luft oberhalb des möglicherweise angesammelten Sekretes in die Trachea zugeführt. Die Luft kann über die natürlichen Atemwege geführt werden, ohne durch möglicherweise vor dem distalen Einlass der Absaugleitung angesammeltes Sekret hindurchströmen zu müssen. Darüber hinaus erlaubt die im Vergleich proximal zu dem distalen Einlass der Absaugleitung angeordnete distale Öffnung der Luftleitung, dass Sekret während des Sprechens abgesaugt werden kann, ohne die Sprachqualität zu beeinträchtigen.

Genau wie die Absaugleitung, kann das Lumen der mindestens einen Luftleitung durch die Halbschale und die Außenseite des Kanülenrohrs begrenzt sein. Auf diese Weise wird das Lumen der Luftleitung durch eine auf das Kanülenrohr dicht aufgesetzte Halbschale und die Außenseite des Kanülenrohrs gebildet.

Die Absaugleitung und/oder die Luftleitung kann bzw. können auch ganz oder teilweise in die Wand des Kanülenrohrs integriert sein.

Das proximale Ende der Absaugleitung kann einen Anschluss zum Anschließen an eine Absaugvorrichtung und das proximale Ende der Luftleitung kann einen Anschluss zum Anschließen an eine Luftzuführvorrichtung aufweisen.

In einer Ausführungsform weist das Kanülenrohr ein Phonationsfenster und/oder eine Siebung auf. Vorzugsweise ist das Phonationsfenster und/oder die Siebung im Bereich der Engstelle der Absaugleitung angeordnet.

Weitere Vorteile, Merkmale und Anwendungsmöglichkeiten der vorliegenden Erfindung werden anhand der vorliegenden Beschreibung einer Ausführungsform und der dazugehörigen Figuren deutlich, wobei gleiche Bezugszeichen auf gleiche Elemente verweisen. Es zeigen:
- Figur 1: eine rückwärtige Ansicht auf eine Beatmungsvorrichtung gemäß einer Ausführungsform der vorliegenden Erfindung,
- Figur 2: eine Seitenansicht auf die Beatmungsvorrichtung gemäß Figur 1,
- Figur 3: eine perspektivische Seitenansicht der Halbschale aus den Figuren 1 und 2, und
- Figur 4: eine Draufsicht auf die Unterseite der Halbschale aus Figur 3.

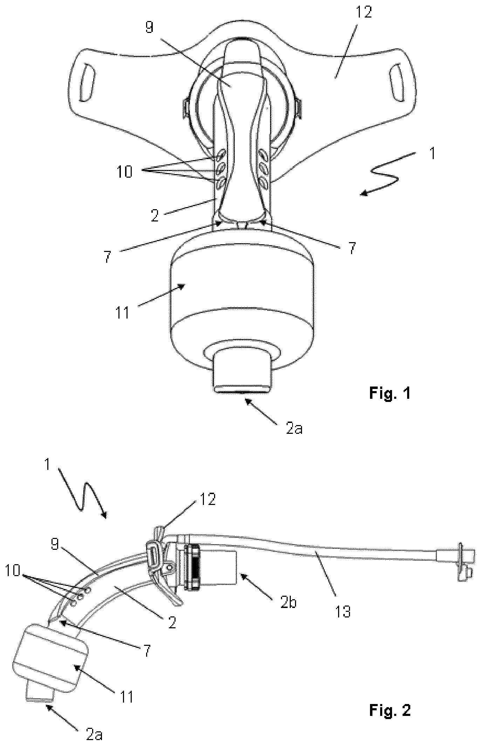

In den Figuren 1 und 2 ist eine erfindungsgemäße Beatmungsvorrichtung 1 einmal in einer rückwärtigen Ansicht (Figur 1) und einmal in einer Seitenansicht (Figur 2) dargestellt. Die Beatmungsvorrichtung 1 weist ein Kanülenrohr 2 mit einer distalen Öffnung 2a und einer proximalen Öffnung 2b auf. Im Bereich des proximalen Ende des Kanülenrohrs 2 ist ein Kanülenschild 12 zur Befestigung der Beatmungsvorrichtung 1 an einem Kanülenband vorgesehen. In der Nähe des distalen Endes des Kanülenrohrs 2 ist ein Cuff 11 zum Abdichten gegenüber der Trachea eines Patienten angeordnet.

Zum Absaugen von Sekret aus dem subglottischen Raum weist das Kanülenrohr 2 eine subglottische Absaugleitung 3 auf, deren Lumen von einer auf die Außenfläche des Kanülenrohrs 2 aufgesetzten Halbschale 9 und der Außenfläche des Kanülenrohrs 2 gebildet wird. Die Absaugleitung 3 befindet sich unter der Halbschale 9 und ist den Figuren 1 und 2 bis auf den distalen Einlass 3a, der durch zwei schlitzförmige Einlässe 7a gebildet wird, nicht zu erkennen. Die Außenfläche des Kanülenrohrs könnte zur Vergrößerung des Querschnitts der Absaugleitung auch eine im Bereich der Absaugleitung axial verlaufende, nutartige Vertiefung aufweisen, die mit der Halbschale 9 die Absaugleitung 3 bildet.

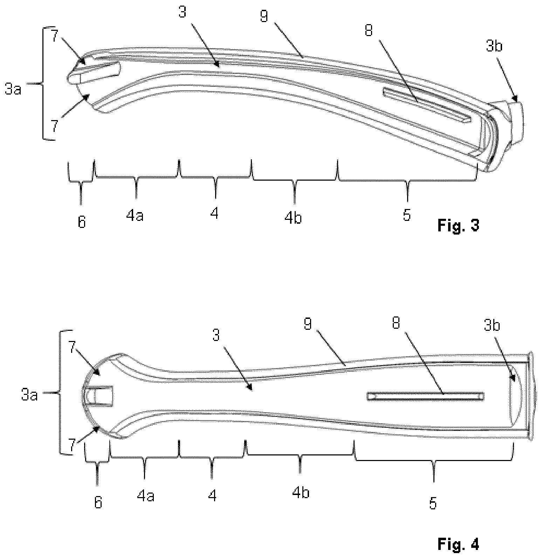

Ein perspektivische Ansicht der Halbschale 9 ist in der Figur 3 und eine Draufsicht auf die Unterseite der Halbschale 9 ist der Figur 4 gezeigt. Die Figuren 3 und 4 zeigen Details der Absaugleitung 3.

Die Absaugleitung 3 hat einen distalen Einlass 3a und einen proximalen Auslass 3b. Sekret aus dem subglottischen Raum kann durch den Einlass 3a in die Absaugleitung 3 eingesaugt und durch den Auslass 3b aus der Absaugleitung 3b herausgeführt werden. Der proximale Auslass 3b kann zu diesem Zweck über einen Absaugschlauch 13 mit einer Absaugvorrichtung verbunden werden, wie es in der Figur 2 angedeutet ist. Der Absaugschlauch 13 ist nicht Teil der Absaugleitung 3.

Die Absaugleitung 3 weist eine Engstelle 4 mit einem minimalen Innenquerschnitt auf. Der Querschnitt der Engstelle 4 ist deutlich kleiner als der maximale Querschnitt der Absaugleitung 3 in den proximalen und distalen Bereichen, die von dem Einlass 3a bzw. dem Auslass 3a und der Engstelle 4 begrenzt sind.

Genauer schließt sich an die Engstelle 4 ein distaler Übergangsbereich 4a an, in dem sich der Querschnitt, beginnend von dem minimalen Querschnitt der Engstelle in Richtung des distalen Einlasses 3a, kontinuierlich auf etwa das 1,5-fache erweitert.

Auf der proximalen Seite der Engstelle schließt sich ein zweiter Bereich 4b an, dessen Innenquerschnittsfläche, ausgehend von dem minimalen Querschnitt der Engstelle 4, kontinuierlich in Richtung des proximalen Auslasses 3b um das 1,18- bis 2,5-fache erweitert. Der zweite Bereich 4b ist 2,4-mal bis 6-mal länger als der erste Bereich 4a.

Sekret, welches durch den distalen Einlass 3a eingesaugt wird, wird aufgrund der Engstelle auf einen kleineren Querschnitt eingeschränkt und beschleunigt, wobei sich beim Passieren der Engstelle Scherkräfte ausbilden, die zähes Sekret oder zusammenhaftende Agglomerationen, auseinanderziehen und zerteilen. Das Sekret kann auf diese Weise einfacher durch die Absaugleitung 3 abgeführt werden.

Damit keine groben Partikel in die Absaugleitung 3 gelangen, weist der distale Einlass 3a mindestens zwei schlitzförmige Öffnungen 7 auf. Der Querschnitt der mindestens zwei Öffnungen 7 ist zusammen so groß wie der maximale Querschnitt der Engstelle 4 am distalen Ende. Der erste Bereich 4a grenzt unmittelbar an den distalen Einlass 3a an.

Weiter weist die Absaugleitung 3 mindestens einen sich in das Lumen der Absaugleitung 3 hineinerstreckenden Vorsprung 8 auf. Der Vorsprung 8 ist bei dieser Ausführungsform ein Steg, der sich in axialer Richtung des Kanülenrohrs 2 erstreckt und das durch die Absaugleitung 3 strömende Sekret zerteilt. Der Vorsprung bzw. Steg 8 könnte sich auch über die gesamte Höhe der Absaugleitung erstecken und effektiv die Halbschale 9 mit der Außenseite des Kanülenrohres 2 verbinden und somit die Absaugleitung 3 in zwei Stränge aufteilen, die der proximalen Seite erst hinter dem Vorsprung 8 wieder zusammengeführt werden. Der Steg 8 stabilisiert darüber hinaus die Absaugleitung 3 und hilft ein Zusammendrücken der Absaugleitung 3 zu verhindern.

Die Beatmungsvorrichtung 1 weist außerdem eine Phonationsöffnung 10 auf.

Das Kanülenschild 12, der Cuff 11, die Phonationsöffnung 10 und die Absaugvorrichtung 13 sind lediglich für eine anschauliche Darstellung der Erfindung beschrieben. Die Erfindung ist grundsätzlich von dem Kanülenschild 12, dem Cuff 11, der Phonationsöffnung 10 und der Absaugvorrichtung 13 unabhängig.

Für Zwecke der ursprünglichen Offenbarung wird darauf hingewiesen, dass sämtliche Merkmale, wie sie sich aus der vorliegenden Beschreibung, den Zeichnungen und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartige Kombinationen unmöglich oder sinnlos machen. Auf die umfassende, explizite Darstellung sämtlicher denkbarerer Merkmalskombinationen wird hier nur der Kürze und der Lesbarkeit der Beschreibung wegen verzichtet.

Während die Erfindung im Detail in den Zeichnungen und der vorangehenden Beschreibung dargestellt und beschreiben wurde, erfolgt diese Darstellung und Beschreibung lediglich beispielhaft und ist nicht als Beschränkung des Schutzbereichs gedacht, so wie er durch die Ansprüche definiert wird. Die Erfindung ist nicht auf die offenbarten Ausführungsformen beschränkt.

Abwandlungen der offenbarten Ausführungsformen sind für den Fachmann aus den Zeichnungen, der Beschreibung und den beigefügten Ansprüchen offensichtlich. In den Ansprüchen schließt das Wort "aufweisen" nicht andere Elemente oder Schritte aus, und der unbestimmte Artikel "eine" oder "ein" schließt eine Mehrzahl nicht aus. Die bloße Tatsache, dass bestimmte Merkmale in unterschiedlichen Ansprüchen beansprucht sind, schließt ihre Kombination nicht aus. Bezugszeichen in den Ansprüchen sind nicht als Beschränkung des Schutzbereichs gedacht.

### Bezugszeichenliste

- 1: Beatmungsvorrichtung
- 2: Kanülenrohr
- 2a: distales Ende des Kanülenrohrs
- 2b: proximales Ende des Kanülenrohrs
- 3: subglottische Absaugleitung
- 3a: distaler Einlass
- 3b: proximaler Auslass
- 4: Engstelle
- 4a: erster Bereich
- 4b: zweiter Bereich
- 5: proximal an den zweiten Bereich anschließender Leitungsabschnitt
- 6: distal an den ersten Bereich anschließender Leitungsabschnitt
- 7: Öffnungen des distalen Einlasses
- 8: Vorsprung
- 9: Halbschale
- 10: Phonationsöffnung/Siebung
- 11: Cuff
- 12: Kanülenschild
- 13: Absaugschlauch

## Patentansprüche

1. Beatmungsvorrichtung (1), insbesondere Tracheostomiekanüle oder Endotrachealtubus, mit einem Kanülenrohr (2), welches eine Absaugleitung (3) zum Absaugen von subglottischem Sekret aufweist, mit einem distalen Einlass (3a) und einem proximalen Auslass (3b), **dadurch gekennzeichnet, dass**
die Absaugleitung (3) im Abstand von dem Einlass (3a) und dem Auslass (3b) mindestens eine Engstelle (4) mit lokal minimalem Querschnitt aufweist, von welcher aus sich der Querschnitt sowohl in proximaler als auch distaler Richtung trichterförmig oder stufenförmig erweitert.

2. Beatmungsvorrichtung (1) nach Anspruch 1, **dadurch gekennzeichnet, dass** sie im Abstand zu dem distalen Einlass (3a) und dem proximalen Auslass (3b) genau eine Engstelle aufweist.

3. Beatmungsvorrichtung (1) nach einem der vorstehenden Ansprüche, **dadurch gekennzeichnet, dass** der minimale Querschnitt an der Engstelle (4) zwischen einschließlich 40 % und einschließlich 85 %, vorzugsweise zwischen einschließlich 50 % und einschließlich 75 %, des maximalen Querschnitts der Absaugleitung proximal und distal von der Engstelle (4) beträgt, und/oder die maximalen Querschnitte in den Bereichen distal und proximal von der Engstelle (4) in etwa gleich groß sind.

4. Beatmungsvorrichtung (1) nach einem der vorstehenden Ansprüche, **dadurch gekennzeichnet, dass** der Querschnitt der Absaugleitung (3), ausgehend von dem minimalen Querschnitt der Engstelle (4), sich in Richtung des distalen Einlasses (3a) in axialer Richtung des Kanülenrohrs (2) über eine Länge von 0,5 cm bis 2,5 cm, vorzugsweise von 0,5 cm bis 1,5 cm, und/oder in Richtung des proximalen Auslasses (3b) in axialer Richtung des Kanülenrohrs (2) über eine Länge von 2 cm bis 8 cm, vorzugsweise von 3 cm bis 6 cm, kontinuierlich oder stufenförmig erweitert.

5. Beatmungsvorrichtung (1) nach einem der vorstehenden Ansprüche, **dadurch gekennzeichnet, dass** der Abstand der Engstelle (4) zum proximalen Auslass (3b) der Absaugleitung (3) größer als der Abstand der Engstelle (4) zum distalen Einlass (3a) der Absaugleitung (3), vorzugsweise um das 2 bis 20-fache größer ist.

6. Beatmungsvorrichtung (1) nach einem der vorstehenden Ansprüche, **dadurch gekennzeichnet, dass** der Abstand der Engstelle (4) von dem distalen Einlass (3a) mindestens 5 mm und von dem proximalen Auslass (3b) mindestens 40 mm beträgt.

7. Beatmungsvorrichtung (1) nach einem der vorstehenden Ansprüche, **dadurch gekennzeichnet, dass** die Engstelle (4) eine axiale Ausdehnung von maximal 5 mm hat, wobei vorzugsweise die Engstelle (4) eine axiale Ausdehnung von mindestens 0,01 mm hat.

8. Beatmungsvorrichtung (1) nach einem der vorstehenden Ansprüche, **dadurch gekennzeichnet, dass** der distale Einlass (3a) durch einen Steg in zwei Öffnungen (7) geteilt ist, wobei der Querschnitt der mindestens zwei Öffnungen (7) zusammen größer als der maximale ungeteilte Querschnitt des sich proximal an den distalen Einlass (3a) anschließenden Abschnittes der Absaugleitung (3) ist.

9. Beatmungsvorrichtung (1) nach einem der vorstehenden Ansprüche, **dadurch gekennzeichnet, dass** die Innenfläche der Absaugleitung (3) außerhalb der Engstelle (4) mindestens einen Vorsprung (8) aufweist, der in das Lumen der Absaugleitung (3) hineinragt, wobei der Vorsprung (8) vorzugsweise ein sich in axialer Richtung der Absaugleitung (3) erstreckender Steg oder Wandabschnitt ist, der bevorzugt im proximalen Bezugsbereich angeordnet ist und/oder das Lumen der Absaugleitung (3) eine in etwa sichelförmige Querschnittsform hat.

10. Beatmungsvorrichtung (1) nach einem der vorstehenden Ansprüche, **dadurch gekennzeichnet, dass** das Lumen der Absaugleitung (3) durch eine auf die Außenseite des Kanülenrohrs (2) aufgesetzte Halbschale (9) und die Außenseite des Kanülenrohrs (2) gebildet wird, und/oder die Außenseite des Kanülenrohres im Bereich der Absaugleitung eine in Längsrichtung des Rohres verlaufende, nutartige Vertiefung aufweist

11. Beatmungsvorrichtung (1) nach einem der vorstehenden Ansprüche, **dadurch gekennzeichnet, dass** mindestens eine weitere Engstelle im Abstand zu dem Einlass und Auslass sowie zu der erstgenannten Engstelle vorgesehen ist, die vorzugsweise durch einen aufgeteilten Querschnitt gebildet wird

12. Beatmungsvorrichtung (1) nach einem der vorstehenden Ansprüche **dadurch gekennzeichnet, dass** die Beatmungsvorrichtung (1) zusätzlich zur Absaugleitung (3) eine Luftleitung mit einer distalen Öffnung und einer proximalen Öffnung aufweist, wobei die distale Öffnung der Luftleitung proximal im Abstand zu dem distalen Einlass (3a) der Absaugleitung (3) angeordnet ist, wobei der minimale lichte Abstand zwischen der distalen Öffnung der Luftleitung und dem distalen Einlass (3a) der Absaugleitung vorzugsweise 5 mm bis 30 mm beträgt.

13. Beatmungsvorrichtung (1) nach einem Anspruch 12, **dadurch gekennzeichnet, dass** das Lumen der Luftleitung durch eine auf das Kanülenrohr (2) dicht aufgesetzte Halbschale (9) und die Außenseite des Kanülenrohrs (2) gebildet wird.

14. Beatmungsvorrichtung (1) nach einem der Ansprüche 1 bis 13, **dadurch gekennzeichnet, dass** die Absaugleitung (3) und/oder die Luftleitung in die Wand des Kanülenrohrs (2) integriert ist bzw. sind.

15. Beatmungsvorrichtung (1) nach einem der Ansprüche 1 bis 14, **dadurch gekennzeichnet, dass** der distale Einlass (3a) mindestens zwei Öffnungen aufweisen, wobei die mindestens zwei Öffnungen vorzugweise zusammen einen Öffnungsquerschnitt aufweisen der dem maximalen Querschnitt des distal zu der Engstelle (4) angeordneten Bereichs der Absaugleitung (3) entspricht, wobei die mindestens zwei Öffnungen des distalen Einlasses (3a) vorzugsweise in unterschiedlichen Ebenen angeordnet sind, wobei die Ebenen vorzugsweise einen Winkel zwischen 10° und 120° miteinander einschließen.

## Claims

1. A ventilator device (1), in particular a tracheostomy cannula or endotracheal tube, comprising a cannula tube (1) which has a suction line (3) for sucking away subglottic secretion, comprising a distal inlet (3a) and a proximal outlet (3b),
**characterised in that**
the suction line (3) at a spacing from the inlet (3a) and the outlet (3b) has at least one constriction (4) with a locally minimum cross-section, from which the cross-section enlarges in a funnel shape or step-wise both in the proximal and also the distal directions.

2. A ventilator device (1) according to claim 1 **characterised in that** it has precisely one constriction at a spacing from the distal inlet (3a) and the proximal outlet (3b).

3. A ventilator device (1) according to one of the preceding claims **characterised in that** the minimum cross-section at the constriction (4) is between 40% and 85% inclusive, preferably between 50% and 75% inclusive, of the maximum cross-section of the suction line proximally and distally from the constriction (4) and/or the maximum cross-sections in the regions distally and proximally from the constriction (4) are approximately of equal size.

4. A ventilator device (1) according to one of the preceding claims **characterised in that** the cross-section of the suction line (3) starting from the minimum cross-section of the constriction (4) enlarges continuously or step-wise in the direction of the distal inlet (3a) in the axial direction of the cannula tube (2) over a length of 0.5 cm to 2.5 cm, preferably 0.5 cm to 1.5 cm, and/or in the direction of the proximal outlet (3b) in the axial direction of the cannula tube (2) over a length of 2 cm to 8 cm, preferably 3 cm to 6 cm.

5. A ventilator device (1) according to one of the preceding claims **characterised in that** the spacing of the constriction (4) from the proximal outlet (3b) of the suction line (3) is greater than the spacing of the constriction (4) from the distal inlet (3a) of the suction line (3), preferably by 2 to 20 times.

6. A ventilator device (1) according to one of the preceding claims **characterised in that** the spacing of the constriction (4) from the distal inlet (3a) is at least 5 mm and from the proximal outlet (3b) at least 40 mm.

7. A ventilator device (1) according to one of the preceding claims **characterised in that** the constriction (4) has an axial extent of a maximum of 5 mm, wherein preferably the constriction (4) has an axial extent of at least 0.01 mm.

8. A ventilator device (1) according to one of the preceding claims **characterised in that** the distal inlet (3a) is divided into two openings (7) by a web, wherein the cross-section of the at least two openings (7) together is greater than the maximum undivided cross-section of the portion of the suction line (3), that proximally adjoins the distal inlet (3a).

9. A ventilator device (1) according to one of the preceding claims **characterised in that** the inside surface of the suction line (3) outside the constriction (4) has at least one projection (8) which protrudes into the lumen of the suction line (3), wherein the projection (8) is preferably a wall portion or web which extends in the axial direction of the suction line (3) and which is preferably arranged in the proximal reference region and/or the lumen of the suction line (3) is of an approximately crescent-shaped cross-sectional shape.

10. A ventilator device (1) according to one of the preceding claims **characterised in that** the lumen of the suction line (3) is formed by a half-shell portion (9) fitted on to the outside of the cannula tube (2) and the outside of the cannula tube (2) and/or the outside of the cannula tube in the region of the suction line has a groove-like depression extending in the longitudinal direction of the tube.

11. A ventilator device (1) according to one of the preceding claims **characterised in that** there is provided at least one further constriction at a spacing from the inlet and outlet and from the first-mentioned constriction, the further constriction preferably being formed by a divided cross-section.

12. A ventilator device (1) according to one of the preceding claims **characterised in that** in addition to the suction line (3) the ventilator device (1) has an air line with a distal opening and a proximal opening, wherein the distal opening of the air line is arranged proximally at a spacing from the distal inlet (3a) of the suction line (3), wherein the minimum internal spacing between the distal opening of the air line and the distal inlet (3a) of the suction line is preferably 5 mm to 30 mm.

13. A ventilator device (1) according to claim 12 **characterised in that** the lumen of the air line is formed by a half-shell portion (9) tightly fitted on to the cannula tube (2) and the outside of the cannula tube (2).

14. A ventilator device (1) according to one of claims 1 to 13 **characterised in that** the suction line (3) and/or the air line is or are integrated into the wall of the cannula tube (2).

15. A ventilator device (1) according to one of claims 1 to 14 **characterised in that** the distal inlet (3a) has at least two openings, wherein the at least two openings preferably together have an opening cross-section which corresponds to the maximum cross-section of the region of the suction line (3), that is arranged distally of the constriction (4), wherein the at least two openings of the distal inlet (3a) are preferably arranged in different planes, wherein the planes preferably include an angle of between 10° and 120° with each other.

## Revendications

1. Dispositif de respiration (1), en particulier canule de trachéotomie ou tube endotrachéal, comportant un tube de canule (2) qui présente un tuyau d'aspiration (3) destiné à aspirer les sécrétions subglottiques, un orifice d'entrée distal (3a) et un orifice de sortie proximal (3b), **caractérisé en ce que** le tuyau d'aspiration (3) présente, à distance de l'orifice d'entrée (3a) et de l'orifice de sortie (3b), au moins un rétrécissement (4) ayant une section transversale localement minimale, à partir duquel la section transversale s'élargit en forme d'entonnoir ou de gradins tant dans la direction proximale que dans la direction distale.

2. Dispositif de respiration (1) selon la revendication 1, **caractérisé en ce qu'**il présente précisément un rétrécissement à distance de l'orifice d'entrée distal (3a) et de l'orifice de sortie proximal (3b).

3. Dispositif de respiration (1) selon l'une des revendications précédentes, **caractérisé en ce que** la section transversale minimale au niveau du rétrécissement (4) est comprise entre 40 % et 85 %, limites comprises, de préférence entre 50 % et 75 %, limites comprises, de la section transversale maximale du tuyau d'aspiration à distance proximale et distale du rétrécissement (4), et/ou **en ce que** les sections transversales ont une taille approximativement identique dans les zones distale et proximale depuis le rétrécissement (4).

4. Dispositif de respiration (1) selon l'une des revendications précédentes, **caractérisé en ce que** la section transversale du tuyau d'aspiration (3), en partant de la section transversale minimale du rétrécissement (4), s'élargit d'une manière continue ou par paliers dans la direction de l'orifice d'entrée distal (3a) dans la direction axiale du tube de canule (2) sur une longueur de 0,5 cm à 2,5 cm, de préférence de 0,5 cm à 1,5 cm, et/ou dans la direction de l'orifice de sortie proximal (3b) dans la direction axiale du tube de canule (2) sur une longueur de 2 cm à 8 cm, de préférence de 3 cm à 6 cm.

5. Dispositif de respiration (1) selon l'une des revendications précédentes, **caractérisé en ce que** la distance du rétrécissement (4) à l'orifice de sortie proximal (3b) du tuyau d'aspiration (3) est supérieure à la distance du rétrécissement (4) à l'orifice d'entrée distal (3a) du tuyau d'aspiration (3), de préférence 2 à 20 fois plus grande.

6. Dispositif de respiration (1) selon l'une des revendications précédentes, **caractérisé en ce que** la distance du rétrécissement (4) à l'orifice d'entrée distal (3a) est d'au moins 5 mm et à l'orifice de sortie proximal (3b) d'au moins 40 mm.

7. Dispositif de respiration (1) selon l'une des revendications précédentes, **caractérisé en ce que** le rétrécissement (4) présente une extension axiale au maximum de 5 mm, le rétrécissement (4) présentant de préférence une extension axiale d'au moins 0,01 mm.

8. Dispositif de respiration (1) selon l'une des revendications précédentes, **caractérisé en ce que** l'orifice d'entrée distal (3a) est subdivisé en deux ouvertures (7) par une bride, la section transversale des ouvertures (7), au moins au nombre de deux, prises ensemble étant supérieure à la section transversale non subdivisée du tronçon du tuyau d'aspiration (3) qui suit l'orifice d'entrée distal (3a) dans la direction proximale.

9. Dispositif de respiration (1) selon l'une des revendications précédentes, **caractérisé en ce que** la surface intérieure du tuyau d'aspiration (3) présente à l'extérieur du rétrécissement (4) au moins une saillie (8), qui pénètre dans la lumière du tuyau d'aspiration (3), la saillie (8) étant de préférence une tige ou une section de paroi s'étendant dans la direction axiale du tuyau d'aspiration (3), et étant de préférence disposée dans la zone de référence proximale, et/ou la lumière du tuyau d'aspiration (3) présente une forme de section transversale approximativement en croissant.

10. Dispositif de respiration (1) selon l'une des revendications précédentes, **caractérisé en ce que** la lumière du tuyau d'aspiration (3) est formée par une demi-coque (9) placée sur le côté extérieur du tube de canule (2) et par le côté extérieur du tube de canule (2), et/ou **en ce que** le côté extérieur du tube de canule présente dans la zone du tuyau d'aspiration un renfoncement en forme de rainure qui s'étend dans la direction longitudinale du tube.

11. Dispositif de respiration (1) selon l'une des revendications précédentes, **caractérisé en ce qu'**au moins un rétrécissement supplémentaire est prévu à distance de l'orifice d'entrée et de l'orifice de sortie, ainsi que du rétrécissement mentionné en premier, qui est de préférence formé par une section transversale subdivisée.

12. Dispositif de respiration (1) selon l'une des revendications précédentes, **caractérisé en ce que** le dispositif de respiration (1) présente en plus du tuyau d'aspiration (3) un tuyau à air comportant une ouverture distale et une ouverture proximale, l'ouverture distale du tuyau à air étant disposée proximalement à distance du premier orifice d'entrée distal (3a) du tuyau d'aspiration (3), la distance libre minimale entre l'ouverture distale du tuyau à air et l'orifice d'entrée distal (3a) du tuyau d'aspiration étant de préférence de 5 mm à 30 mm.

13. Dispositif de respiration (1) selon la revendication 12, **caractérisé en ce que** la lumière du tuyau à air est formée par une demi-coque (9) placée d'une manière hermétique sur le tube de canule (2) et par le côté extérieur du tube de canule (2).

14. Dispositif de respiration (1) selon l'une des revendications 1 à 13, **caractérisé en ce que** le tuyau d'aspiration (3) et/ou le tuyau à air est(sont) intégré(s) dans la paroi du tube de canule (2).

15. Dispositif de respiration (1) selon l'une des revendications 1 à 14, **caractérisé en ce que** l'orifice d'entrée distal (3a) présente au moins deux ouvertures, les ouvertures, au moins au nombre de deux, présentant de préférence ensemble une section transversale d'ouverture qui correspond à la section transversale maximale de la zone du tuyau d'aspiration (3) disposée distalement par rapport au rétrécissement (4), les ouvertures, au moins au nombre de deux, de l'orifice d'entrée distal (3a) étant de préférence disposées dans des plans différents, les plans faisant entre eux de préférence un angle compris entre 10° et 120°.
